# EUROPEAN PATENT APPLICATION

(11) **EP 4 670 745 A1**
(43) Date of publication of application: **31.12.2025**
(21) Application number: 25163653.6
(22) Date of filing: 13.03.2025
(51) Int. Cl.: A61L 2/10

(54) **PORTABLE STERILIZER FOR A DRINKING CONTAINER**

(30) Priority: 28.06.2024 CN 202421520248 U
(71) Applicant: Sabine IP LLC, Monroe, LA 71203 (US)
(72) Inventor: Guoxiong, Ruan, Fujian, 361026 (CN); Daitong, Li, Fujian, 361026 (CN)
(74) Representative: Barker Brettell LLP

(57) **Abstract**

A portable sterilizer for a drinking container, the sterilizer comprising:
a main shell;
a receiving channel extending vertically in the middle of the main shell, and comprising an upper opening and a lower opening;
an upper shell fixed in the receiving channel and having an upper opening connected to the upper opening of the receiving channel;
a lower shell fixed in the receiving channel and having a lower opening connected to the lower opening of the receiving channel;
a lid disinfection chamber formed by the upper shell and the main shell;
a bottle body disinfection chamber formed by the lower shell and the main shell;
an upper disinfection component fixed with a bottom of the upper shell, the upper disinfection component passing through the upper shell to disinfect the lid disinfection chamber; and
a lower disinfection component fixed with a top of the lower shell, the lower disinfection component passing through the lower shell to disinfect the bottle body disinfection chamber,
wherein the bottom of the upper shell and the top of the lower shell are spaced apart from each other.

## Description

### Field of the Invention

The present invention relates to a portable sterilizer, in particular, but not limited to, a portable sterilizer for a drinking container such as an insulated bottle or a cooling bottle.

### Background to the Invention

Insulated bottles and cooling bottles (also known as insulated cups and cooling cups) are examples of commonly used drinking containers. Insulated bottles and cooling bottles have different characteristics and are used for different functions. The interior of an insulated drinking container, such as a Thermos^{™} bottle or flask, is effectively isolated from the heat exchange of the exterior through the design of a vacuum layer, so that liquid inside the container can maintain a certain temperature for a long time. For example, an insulated bottle can be used to maintain the temperature of hot drinks in the cold winter, or to maintain the coolness of cold drinks in the hot summer. Insulated bottles are provided with good heat preservation properties.

Cooling bottles are an emerging drinking container, whose main function is to quickly reduce the temperature of the liquid. Cooling bottles are constructed using special materials and a structural design that allows the cooling cup to reduce a high-temperature liquid inside the bottle to a suitable drinking temperature in a short period of time, providing a more comfortable drinking experience for the user.

As the frequency of use of drinking containers such as insulated bottles and cooling bottles increases, there is an increasing need to sterilize them. Existing sterilizers are provided with a large sterilization chamber for accommodating and sterilizing a drinking container. An example of a sterilizer is set out in CN220608714U, describing a portable sterilization cabinet having a shell that encloses a sterilization space to sterilize drinking containers such as milk bottles and teacups. Existing sterilizers are large in size, making them inconvenient to use.

### Summary of the Invention

The present invention provides a portable sterilizer for a drinking container that addresses the above problem. In particular, it is an object of the present invention to provide a portable sterilizer to address the problem of conventional sterilizers being large in size and inconvenient to use.

According to an aspect of the present invention, there is provided a portable sterilizer for a drinking container, the sterilizer comprising:
a main shell;
a receiving channel extending vertically in the middle of the main shell, and comprising an upper opening and a lower opening;
an upper shell fixed in the receiving channel and having an upper opening connected to the upper opening of the receiving channel;
a lower shell fixed in the receiving channel and having a lower opening connected to a lower opening of the receiving channel;
a lid disinfection chamber formed by the upper shell and the main shell;
a bottle body disinfection chamber formed by the lower shell and the main shell;
an upper disinfection component fixed with a bottom of the upper shell, the upper disinfection component passing through the upper shell to disinfect the lid disinfection chamber; and
a lower disinfection component fixed with a top of the lower shell, the lower disinfection component passing through the lower shell to disinfect the bottle body disinfection chamber,
wherein the bottom of the upper shell and the top of the lower shell are spaced apart from each other.

The portable sterilizer is provided with two disinfection chambers for disinfecting different parts of the drinking container, so the sterilizer can disinfect from both upper and lower directions. This allows the internal space of the receiving channel to be utilized fully, allowing the effective overall volume of the sterilizer to be reduced and making the sterilizer more convenient to use. The drinking container may include a lid and a body. By providing a portable sterilizer with two disinfection chambers, the lid disinfection chamber may accommodate the lid, while the bottle body disinfection chamber may accommodate the bottle body. The upper disinfection component passes through the upper shell to disinfect the lid, while the lower disinfection component passes through the lower shell to disinfect the bottle body. The lid and the body of the bottle may therefore be disinfected from both the upper and lower directions, making full use of the internal space of the receiving channel. This effectively reduces the overall volume of the sterilizer, improving portability and convenience of use.

The bottom of the upper shell may comprise one or more upper disinfection holes.

A portion of the upper disinfection component passing through the upper shell may correspond with the position(s) of the upper disinfection hole(s). A portion of the upper disinfection component passing through the upper shell may correspond one-to-one with the positions of the upper disinfection holes.

The top of the lower shell may comprise one or more lower disinfection holes.

A portion of the lower disinfection component passing through the lower shell may correspond with the position(s) of the lower disinfection hole(s). A portion of the lower disinfection component passing through the lower shell may correspond one-to-one with the positions of the lower disinfection holes.

The top of the lower shell may comprise at least one inner circle disinfection hole. The top of the lower shell may comprise at least two outer circle disinfection holes. The at least two outer circle disinfection holes may be arranged around the inner circle disinfection hole.

The, or each, outer circle disinfection hole may correspond to an outer side of the drinking container.

The, or each, inner circle disinfection hole may correspond to an inner side of a top of the drinking container.

The upper shell may comprise an upper sensing hole. The upper sensing hole may be open on a top of the upper shell.

The upper disinfection component may comprise an upper sensor. The upper sensor may extend upward through the upper sensing hole.

A top of the receiving channel may comprise a step. The step may be arranged outwardly extending. The step may be a storage step.

A top of the upper shell may be fixedly abutted against a bottom surface of the step.

The step may comprise a sensing through-hole corresponding to the upper sensing hole.

The sensing through-hole may be connected with the upper sensing hole.

The lower shell may comprise a lower sensing hole that is open on a top of the lower shell.

The lower disinfection component may comprise a lower sensor. The lower sensor may extend downward through the lower sensing hole.

The main shell may comprise a buttonhole.

An indicator light button component may be fixed on the buttonhole.

The indicator light button component may be electrically connected to the upper disinfection component. The indicator light button component may be electrically connected to the lower disinfection component. The indicator light button component may be electrically connected to the upper disinfection component and the lower disinfection component.

The main shell may comprise a charging hole.

### Brief Description of the Drawings

Embodiments of the invention are now described, by way of example only, hereinafter with reference to the accompanying drawings, in which:
- **Figure 1**: shows a schematic exploded view of a portable sterilizer;
- **Figure 2**: shows a schematic cross-sectional view of the portable sterilizer;
- **Figure 3**: shows a perspective side view of a main shell of the portable sterilizer;
- **Figure 4**: shows a perspective top view of a lower shell of the portable sterilizer;
- **Figure 5**: shows a perspective top view of an upper shell of the portable sterilizer; and
- **Figure 6**: shows a schematic cross-sectional view of the portable sterilizer in use.

### Detailed Description

Like reference numerals are used to depict like features throughout.

In order to make the purpose, technical solution and advantages of the present invention clearer, the present invention is further described in detail below with reference to the accompanying drawings and embodiments. It should be understood that the specific embodiments described here are only used to explain the present invention and are not limiting.

In addition, it should be noted that the terms "up", "down", "left", "right", "vertical", "horizontal", "inside", "outside", etc. are based on the orientation or position relationship shown in the accompanying drawings, and are only for the convenience of describing the present invention and simplifying the description, rather than indicating or implying that the device or element of the present invention must have a specific orientation, and therefore cannot be understood as a limitation on the present invention.

When an element is referred to as being "fixed to" or "disposed on" or "provided on" another element, it may be directly on the other element or indirectly on the other element. When an element is referred to as being "connected to" another element, it may be directly connected to the other element or indirectly connected to the other element.

Unless otherwise clearly specified and limited, the terms "installed" and "connected" should be understood in a broad sense, for example, it can be a fixed connection, a detachable connection, or an integral connection; it can be a mechanical connection or an electrical connection; it can be a direct connection or an indirect connection through an intermediate medium, it can be the internal connection of two elements or the interaction relationship between two elements.

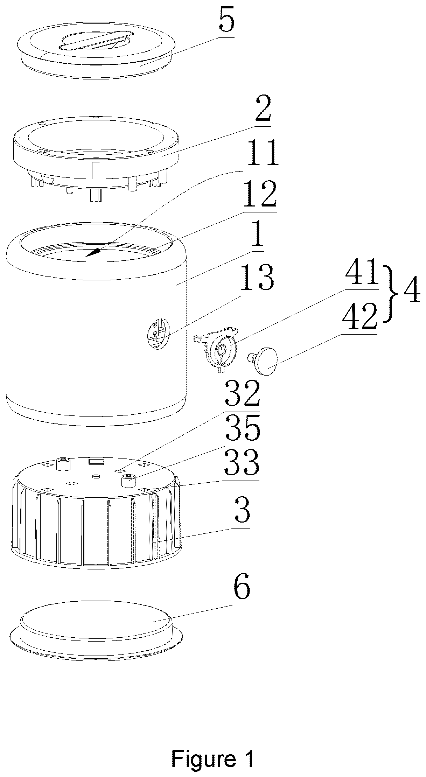

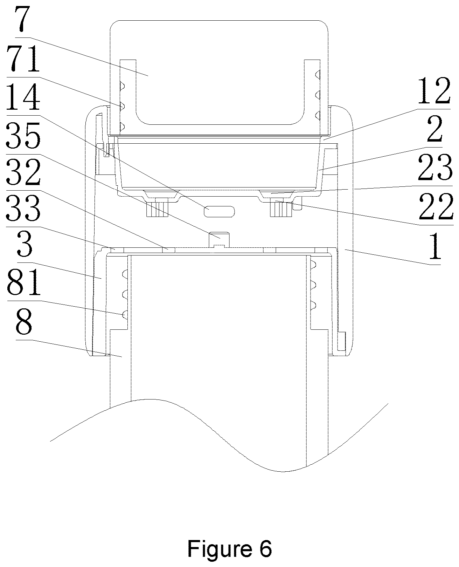

Referring to Figures 1 to 6, there is shown a portable sterilizer for a drinking container such as an insulated bottle or a cooling bottle. The portable sterilizer includes a main shell 1 and a vertically extending receiving channel 11 provided in the middle of the main shell 1. An upper shell 2 and a lower shell 3 are fixed in the receiving channel 11. An upper opening of the upper shell 2 is connected to an upper opening of the receiving channel 11. The upper shell 2 and the main shell 1 are enclosed to form a lid disinfection chamber 21. A lower opening of the lower shell 3 is connected to a lower opening of the receiving channel 11. The lower shell 3 and the main shell 1 are enclosed to form a bottle body disinfection chamber 31.

A bottom of the upper shell 2 and a top of the lower shell 3 are spaced apart from each other, and an upper disinfection component (not shown) and a lower disinfection component (not shown) are respectively fixed. The upper disinfection component passes through the upper shell 2 to disinfect the lid disinfection chamber 21, or a lid of a drinking container placed in the lid disinfection chamber 21. The lower disinfection component passes through the lower shell 3 to disinfect the bottle body disinfection chamber 31, or a body of a drinking container placed in the bottle body disinfection chamber 31.

In use, the lid disinfection chamber 21 accommodates a lid 7 of a drinking container, and the bottle body disinfection chamber 31 accommodates the top of a bottle body 8 of the drinking container. The upper disinfection component passes through the upper shell 2 to disinfect the lid 7, and the lower disinfection component passes through the lower shell 3 to disinfect the bottle body 8. The bottle body 8 and lid 7 of the drinking container are disinfected from the upper and lower directions, making full use of the internal space of the receiving channel 11, effectively reducing the overall volume and therefore improving portability and convenience of use.

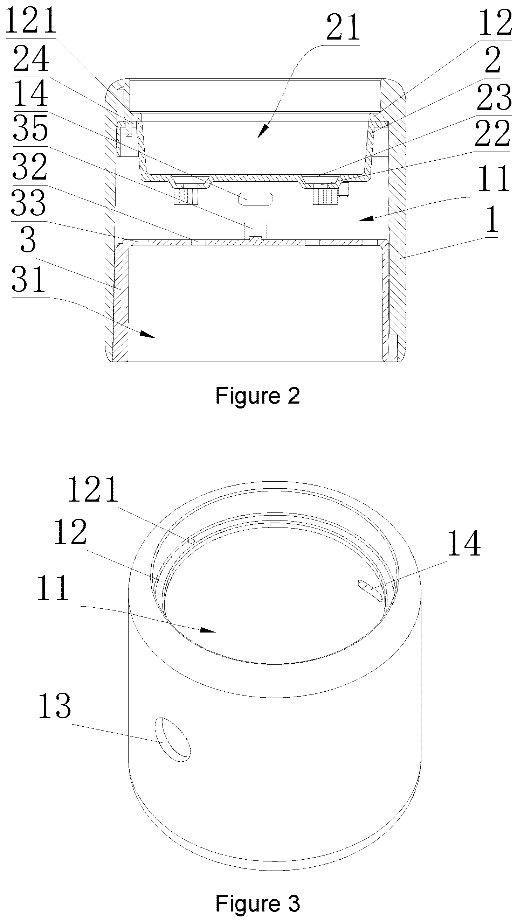

Referring to Figures 2, 5 and 6, the bottom of the upper shell 2 is provided with an upper disinfection hole 22, and the top of the lower shell 3 is provided with a lower disinfection hole group. The disinfection ends of the upper disinfection component and the lower disinfection component correspond to the upper disinfection hole 22 and the lower disinfection hole group position one-by-one. Specifically, the upper disinfection component and the lower disinfection component both include a control circuit board, and the control circuit board is electrically connected with a disinfection lamp, that is, the disinfection lamp of the upper disinfection component corresponds to the upper disinfection hole 22 one-by-one, and the disinfection lamp of the lower disinfection component corresponds to the lower disinfection hole group one-by-one. The disinfection lamp is configured to emit UV light for disinfection during use. Other types of disinfection devices are also envisaged. The lid 7 is located above the upper shell 2, and the disinfection lamp of the upper disinfection component passes through the upper disinfection hole 22 to disinfect the bottom of the lid 7 and the inner internal thread 71.

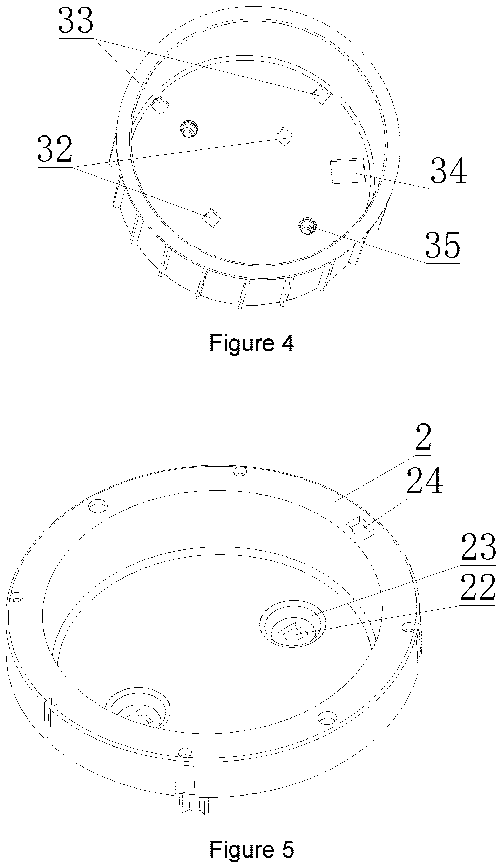

Referring to Figure 4, the lower disinfection hole group includes at least one inner circle disinfection hole 32 and at least two outer circle disinfection holes 33 arranged around the inner circle disinfection hole 32. In this embodiment, the number of the inner circle disinfection holes 32 is two, and the two inner circle disinfection holes 32 are located on the same diameter of the lower shell 3 and are symmetrically arranged with the centre of the circle as the midpoint; the number of the outer circle disinfection holes 33 is four, and the four outer circle disinfection holes 33 are evenly spaced and distributed on the outside of the inner circle disinfection hole 32. Similarly, the inner circle disinfection holes 32 and the outer circle disinfection holes 33 can be set to other numbers according to the disinfection requirements.

In this embodiment, the outer circle disinfection hole 33 corresponds to the outer position of the top of the bottle body 8, and the inner circle disinfection hole 32 corresponds to the inner position of the top of the bottle body 8. The disinfection lamp of the lower disinfection component passes through the inner circle disinfection hole 32 to disinfect the inner wall of the bottle body 8, and the disinfection lamp of the lower disinfection component passes through the outer circle disinfection hole 33 to disinfect the external thread 81 on the outer side of the top of the bottle body 8. When the bottle body 8 is in use, the outer side of the top of the bottle body 8 is in closest contact with the user's mouth, and a plurality of outer circle disinfection lamps are arranged to disinfect the outer side of the top of the bottle body 8 in multiple directions, thereby fully improving the disinfection effect.

In this embodiment, there are two upper disinfection holes 22, and there are also two disinfection lamps in the upper disinfection component, which correspond one-to-one with the two upper disinfection holes 22. The overall disinfection lamp is used in a small number, and the energy consumption is fully reduced while ensuring a good disinfection effect, so that it can be used for multiple disinfection after a single charge, reducing the number of charging times after going out and improving the user experience. In this embodiment, a trumpet-shaped notch 23 is provided at the top of the upper disinfection hole 22, that is, the top of the upper disinfection hole 22 is in the shape of a truncated cone with a large top and a small bottom. The trumpet-shaped notch 23 ensures that the light of the disinfection lamp of the upper disinfection component can be diffused to the surroundings, and the internal thread 71 of the lid 7 is fully irradiated for disinfection and sterilization, further improving the overall disinfection effect.

Referring to Figure 2, an upper sensing hole 24 is provided on the top peripheral side of the upper shell 2. The upper disinfection component further includes an upper sensor, which extends upward through the upper sensing hole 24. The upper sensor is electrically connected to the control circuit board of the upper disinfection component. When the lid 7 is placed, the edge of the lid 7 applies pressure to the upper sensor, thereby controlling the upper disinfection lamp to turn on through the control circuit board of the upper disinfection component. When the lid 7 is lifted, the upper disinfection lamp is turned off to avoid energy loss.

A lower sensing hole 34 is provided at the top of the lower shell 3, and the lower disinfection component further includes a lower sensor, which extends downward through the lower sensing hole 34. The lower sensor is electrically connected to the control circuit board of the lower disinfection component. When the bottle body 8 is placed, the edge of the bottle body 8 contacts the lower sensor, and the lower sensor senses the bottle body 8 through contact. Similarly, the lower sensor can also sense the edge of the bottle body 8 through photoelectric sensing, thereby controlling the lower disinfection lamp to turn on through the control circuit board of the lower disinfection component. When the bottle body 8 is picked up, the lower disinfection lamp is turned off to avoid energy loss.

The upper disinfection lamp and the lower disinfection lamp are controlled by the upper sensor and the lower sensor respectively. Similarly, the lower disinfection lamp can be electrically connected to the control circuit board of the upper disinfection component, and the upper sensor is used to synchronously control the upper and lower disinfection lamps, that is, it is determined that the placement order of the lid 7 and the bottle body 8 is: first the bottle body 8, and then the lid 7. The placement of the lid 7 indicates that the bottle body 8 has been placed, reducing the number of sensors and reducing costs. Similarly, the upper disinfection lamp can be electrically connected to the control circuit board of the lower disinfection component, and the lower sensor is used to synchronously control the upper and lower disinfection lamps, that is, it is determined that the placement order of the lid 7 and the bottle body 8 is: first the lid 7, and then the bottle body 8. The placement of the bottle body 8 indicates that the lid 7 has been placed, reducing the number of sensors and reducing costs.

The top of the receiving channel 11 is provided with a storage step 12 extending outward, and the top peripheral side of the upper shell 2 is fixed against the bottom surface of the storage step 12. In this embodiment, the upper shell 2 is fixedly connected to the storage step 12 by bolts. The storage step 12 is provided with a sensing through hole 121, which corresponds to the upper sensing hole 24 one by one and is connected to each other. The storage step 12 provides a horizontal placement plane for the lid 7 to ensure the stability of the placement of the lid 7. In this embodiment, the top of the lower shell 3 is provided with a mounting threaded column 35 and is fixedly connected to the circuit board or other structure inside the main shell 1 by bolts to ensure the firmness of the installation of the lower shell 3.

Referring to Figure 1, a buttonhole 13 is provided on the main shell 1, and an indicator light button component 4 is fixed on the buttonhole 13. The indicator light button component 4 is electrically connected to the upper disinfection component and the lower disinfection component. In the present embodiment, the indicator light button component 4 includes an indicator light holder 41 and a button 42, and the indicator light holder 41 is fixed on the buttonhole 13. The indicator light holder 41 is a translucent material component, and the button 42 extends inward through the indicator light holder 41.

In the present embodiment, switches are provided on the control circuit boards of the upper disinfection component and the lower disinfection component, and the button 42 is electrically connected to the switch on the control circuit board to control the on and off of the switch. The indicator light holder 41 is electrically connected to the control circuit board, and the light of the indicator light on the control circuit board radiates outward through the indicator light holder 41 to indicate the disinfection working state.

Referring to Figure 3, a charging hole 14 is opened on the main shell 1, and charging ports and batteries are provided on the control circuit boards of the upper disinfection component and the lower disinfection component. A charging interface (not shown) is fixed in the charging hole 14, and the charging interface is electrically connected to the charging port to charge the sterilizer.

Referring to Figure 1, an upper cover 5 and a lower cover 6 are respectively provided on the openings of the upper shell 2 and the lower shell 3. The upper cover 5 and the lower cover 6 seal and protect the lid disinfection chamber 21 and the bottle body disinfection chamber 31 to prevent dust from entering and affecting the stability of the sterilizer.

It will be appreciated by persons skilled in the art that the above embodiment(s) have been described by way of example only and not in any limitative sense, and that various alterations and modifications are possible without departing from the scope of the invention as defined by the appended claims.

### List of features and reference numerals

- 1: Main shell
- 2: Upper shell
- 3: Lower shell
- 4: Indicator light button component
- 5: Upper lid
- 6: Lower lid
- 7: Lid
- 8: Bottle body
- 11: Accommodation channel
- 12: Storage step
- 13: Buttonhole
- 14: Charging hole
- 21: Lid disinfection chamber
- 22: Upper disinfection hole
- 23: Trumpet-shaped notch
- 24: Upper sensing hole
- 31: Bottle body disinfection chamber
- 32: Inner ring disinfection hole
- 33: Outer ring disinfection hole
- 34: Lower induction hole
- 35: Installation threaded column
- 41: Indicator light socket
- 42: Button
- 71: Internal thread
- 81: External thread
- 121: Sensing through hole

## Claims

1. A portable sterilizer for a drinking container, the sterilizer comprising:
a main shell;
a receiving channel extending vertically in the middle of the main shell, and comprising an upper opening and a lower opening;
an upper shell fixed in the receiving channel and having an upper opening connected to the upper opening of the receiving channel;
a lower shell fixed in the receiving channel and having a lower opening connected to a lower opening of the receiving channel;
a lid disinfection chamber formed by the upper shell and the main shell;
a bottle body disinfection chamber formed by the lower shell and the main shell;
an upper disinfection component fixed with a bottom of the upper shell, the upper disinfection component passing through the upper shell to disinfect the lid disinfection chamber; and
a lower disinfection component fixed with a top of the lower shell, the lower disinfection component passing through the lower shell to disinfect the bottle body disinfection chamber,
wherein the bottom of the upper shell and the top of the lower shell are spaced apart from each other.

2. A portable sterilizer according to claim 1, wherein the bottom of the upper shell comprises one or more upper disinfection holes.

3. A portable sterilizer according to claim 2, wherein a portion of the upper disinfection component passing through the upper shell corresponds with the position(s) of the upper disinfection hole(s).

4. A portable sterilizer according to any preceding claim, wherein the top of the lower shell comprises one or more lower disinfection holes.

5. A portable sterilizer according to claim 4, wherein a portion of the lower disinfection component passing through the lower shell corresponds with the position(s) of the lower disinfection hole(s).

6. A portable sterilizer according to claim 4 or claim 5, wherein the top of the lower shell comprises at least one inner circle disinfection hole and at least two outer circle disinfection holes arranged around the inner circle disinfection hole.

7. A portable sterilizer according to claim 6, wherein the, or each, outer circle disinfection hole corresponds to an outer side of the drinking container.

8. A portable sterilizer according to claim 6 or claim 7, wherein the, or each, inner circle disinfection hole corresponds to an inner side of a top of the drinking container.

9. A portable sterilizer according to any preceding claim, wherein the upper shell comprises an upper sensing hole that is open on a top of the upper shell.

10. A portable sterilizer according to claim 9, wherein the upper disinfection component comprises an upper sensor extending upward through the upper sensing hole.

11. A portable sterilizer according to claim 9 or claim 10, wherein a top of the receiving channel comprises an outwardly extending step.

12. A portable sterilizer according to claim 11, wherein a top of the upper shell is fixedly abutted against a bottom surface of the step.

13. A portable sterilizer according to claim 12, wherein the step comprises a sensing through-hole corresponding to the upper sensing hole.

14. A portable sterilizer according to claim 13, wherein the sensing through-hole is connected with the upper sensing hole.

15. A portable sterilizer according to any preceding claim, wherein the lower shell comprises a lower sensing hole that is open on a top of the lower shell.

16. A portable sterilizer according to claim 15, wherein the lower disinfection component comprises a lower sensor extending downward through the lower sensing hole.

17. A portable sterilizer according to any preceding claim, wherein the main shell comprises a buttonhole.

18. A portable sterilizer according to claim 17, wherein an indicator light button component is fixed on the buttonhole.

19. A portable sterilizer according to claim 18, wherein the indicator light button component is electrically connected to the upper disinfection component and the lower disinfection component.

20. A portable sterilizer according to any preceding claim, wherein the main shell comprises a charging hole.
